(19) Europäisches Patentamt
European Patent Office
Office européen des brevets

(11) **EP 2 168 505 A1**

(12) **EUROPÄISCHE PATENTANMELDUNG**

(43) Veröffentlichungstag:
**31.03.2010 Patentblatt 2010/13**

(51) Int Cl.:
***A61B 17/16*** *(2006.01)*

(21) Anmeldenummer: 07023956.1

(22) Anmeldetag: **11.12.2007**

(84) Benannte Vertragsstaaten:
**AT BE BG CH CY CZ DE DK EE ES FI FR GB GR HU IE IS IT LI LT LU LV MC MT NL PL PT RO SE SI SK TR**
Benannte Erstreckungsstaaten:
**AL BA HR MK RS**

(30) Priorität: **12.12.2006 AT 20472006**

(71) Anmelder: **Strehl, Bernhard**
**8055 Neu-Seiersberg (AT)**

(72) Erfinder: **Strehl, Bernhard**
**8055 Neu-Seiersberg (AT)**

Bemerkungen:
Die Patentansprüche 16-17 gelten als fallen gelassen, da die entsprechenden Anspruchsgebühren nicht entrichtet wurden (R. 45 (3) EPÜ).

(54) **Instrument zur Anfertigung von Öffnungen in Knochen in Form eines Knochendeckels**

(57)　Die Erfindung bezieht sich auf eine Vorrichtung zur Anfertigung von Öffnungen in Knochen in Form eines Knochendeckels. Die Besonderheit der vorliegenden Erfindung liegt darin, dass mehrere Schneiddornen (4a, 4b, 4c, 4d, 4e) entlang einer Hypotrochoide geführt werden und so mit dieser Vorrichtung unter Zuhilfenahme eines rotierenden Antriebes Öffnungen und Deckel in einer Form anfertigbar sind, die gleichseitigen Polygonen mit abgerundeten Ecken ähneln.

　Die Vorrichtung besteht aus einem innenverzahnten Hohlzylinder (1) und mehreren außenverzahnete Zahnrädern (3a, 3b, 3c, 3d, 3e) mit den Schneiddornen (4a, 4b, 4c, d, 4e).

Figur 4

Printed by Jouve, 75001 PARIS (FR)

**Beschreibung**

**Hintergrund der Erfindung**

Es wird eine Vorrichtung zur Anfertigung von Öffnungen in Knochen in Form eines Knochendeckels beschrieben, die unter Zuhilfenahme eines rotierenden Antriebes diese Öffnungen und Deckel in einer Form erzeugt, die nahezu gleichseitigen Polygon n ähneln. Eine solche Knochenfräse bzw. Knochensäge lässt sich überall dort vorteilhaft ein etzen, wo eine Öffnung in einen knöchernen Hohlraum durch Entfernen eines kompakten Knochenstückes (dem so genannten Knochendeckel) hergestellt werden soll, und die Öffnung später mit dem entfernten Knochenstück wieder verschlossen werden soll.

Für die Herstellung von Knochenöffnungen und Knochendeckeln sind eine Vielzahl von Vorrichtungen bekannt (z.B. DE4029676A1, EP0962192A1, GB110179A, GB 8941420A, GB1455566A, GB2420979A, US4768504A1, US5201749A1, W09710765A1). Diese können im Wesentlichen in folgende vier Gruppen eingeteilt werden:

A) Kleine Kreissägen, bei denen das Sägeblatt entweder rotiert oder dreh-oszillien und mit der Hand geführt werden. Mit Hilfe dieser Kreissägen lassen sich gerade Schni te in den

Knochen setzen. Um eine Knochenöffnung bzw. einen Knochendeckel anzufertigen müssen mehrere gerade Schnitte durchgeführt werden, die üblicherweise in Form eines Bechteckes angeordnet werden.

B) Kleine oszillierende Sticksägen, die ebenfalls von Hand geführt werden. Mit Hilfe eines auf- und aboszillierenden Sägeblattes können Konturen bis zu einem Minimalradius in den

Knochen gesägt werden. Zum erstmaligen Durchdringen des Knochens wird ent veder ein kleines Loch vorgebohrt, in das die oszillierende Stichsäge dann eingeführt wird oder die Stichsäge wird in sehr flachen Winkel schräg an die Knochenoberfläche angesetzt und raspelt sich dann durch den Knochen durch. Nach dem erstmaligen Durchdringen durch den Knochen wird die Stichsäge senkrecht gestellt, um danach die gewünschte Konturauszusägen zu können.

C) Kreisrunde Lochsägen, bei denen die Zähne der Säge axial entlang eines Kreisbogens angeordnet sind und die um einen Zentrierstift rotieren. Mit Hilfe dieser Lochsäge i werden exakt kreisrunde Ausnehmungen und Deckel erzielt. Manche Ausführungen solcher

Knochensägen verfügen über einen Zentrierstift, der in eine vorgebohrte Zentrierbohrung eingeführt wird.

D) Mit Ultraschall schwingende Aufsätze, die durch Zerreiben bzw. Abreiben den Knochen kleinweise abtragen und mit denen so Ausnehmungen in den Knochen gearbeitet werden. Die

Form der Öffnung ist dabei entweder durch die Form des Ultraschallaufsatzes definiert, oder der Ultraschallaufsatz ist schneidenförmig oder fräserartig ausgeführt und wird mit der Hand entlang der gewünschten Kontur geführt. Die Oberflächen der Ultraschallauf ätze sind üblicherweise mit einem Schleifmittel beschichtet oder mit einer Vielzahl von kleinen

Schneidkanten versehen.

Die Verwendung der unter A) genannten Kreissägen hat folgende Nachteile:

• An den Eckpunkten des Rechteckes muss über die Länge der Seite des Rechteckes hinaus geschnitten werden, da nur so der Knochen in der vollen Dicke durchtrennt werden kann. Daher weist die Schnittlinie an den Eckpunkten Kreuze auf md mehr Knochen, als notwendig, muss durchtrennt werden.

• Das Kreissägeblatt muss tiefer, als die Knochendicke eingeschoben werden, um einen guten Schnitt zu erhalten, insbesonders an den Eckpunkten. Dadurch besteht die Gefahr, das Gewebestrukturen, die unmittelbar unterhalb des Knochens liegen, eschädigt werden.

• Da die Drehachse der Kreissäge parallel zur Knochenoberfläche ist und der Antriebsmechanismus Platz benötigt, ist die Kreissäge speziell in beengten Situationen nur schwer zu führen. Oft können die Knochendeckel nicht an der Stelle gesetzt werden, wo sie idealerweise gesetzt werden sollten.

Die Verwendung der unter B) genannten oszillierenden Stichsägen hat folgende Nachteile:

• Das für das Einfädeln der Stichsäge erforderlich vorgebohrte Loch muss rela iv großes sein, was einen großen Knochendefekt bedeutet Das schräge Ansetzen der St chsäge in flachen Winkel ist bei unebener Knochenoberfläche nicht durchführbar und erfordert auf jeden Fall sehr viel Platz.

• Die Tiefe, bis in die die Spitze des Stichsägeblattes oszilliert, kann nur seiner schwer kontrolliert werden und es besteht die Gefahr, das Gewebestrukturen, die unmittelbar unterhalb des Knochens liegen, geschädigt werden.

• Die Radien der auszusägenden Kontur können einen Minimalradius nicht unterschreiten, der durch die Geometrie des Stichsägeblattes und die 1 reite des Schnittkanal bestimmt ist.

Die Verwendung der unter C) genannten Lochsägen hat folgende Nachteile:

- Die erzielte Öffnung und der Knochendeckel sind exakt kreisrund. Da lurch den Sägevorgang Knochenmaterial entfernt wird, ist der Durchmesser der Knochenöffnung größer, als jener des verbleibenden Knochendeckels. Dadurch ergibt sich beim Wiedereinsetzen des Knochendeckels eine sichelförmige Öffnung und der Knochendeckel liegt nur an einer Stelle am Rand der Knochenöffnung an. Dadurch ist die Einheilung des Knochendeckels in vielen Fällen erschwert.

Die Verwendung der unter D) genannten Ultraschallaufsätze hat folgende Nachte le:

- Das Arbeiten geht sehr langsam von Statten. Die Schneidkanten werden lurch das Knochengewebe und durch das umgebende Weichgewebe verklebt und schneiden dann nicht mehr.

Beschreibung

Der Erfindung liegt die Aufgabe zugrunde, Öffnungen in Knochen in form von Knochendeckeln herzustellen und dabei die Form der Kontur der Öffnung so zugestalten, dass auch nach dem unvermeidlichen Materialverlust durch den Sägevorgang, der Knochendeckel beim Wiedereinsetzen in die Öffnung nicht nur punktförmig am Rand der Öffnung anliegt, sondern an einer möglichst langen kontinuierlichen Kante. Zusätzlich soll rasch gearbeitet werden können. Die Tiefe, bis in die die Vorrichtung unter den Knochen eindringt, soll erstens kontrolliert werden können und zweitens möglichst gering sein. Schließlich soll die Vorrichtung von einem rotierenden Antrieb angetrieben werden, wie er im chirurgi chen und zahnärztlichen Bereich häufig verwendet wird.
Dies wird durch folgende Merkmale erreicht:

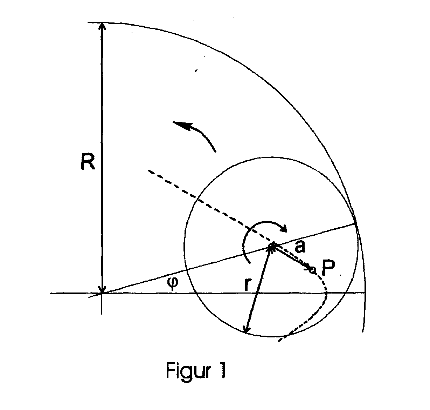

1. Die Erzeugung des Schnittkanals erfolgt dadurch, dass mehrere Schneiddornen entlang einer Hypotrochoide um eine zentrale Achse geführt werden.
2. Die Hypotrochoide wird dabei durch folgende Gleichungen und Parameter beschrieben (siehe auch Fig. 1):

$$x(\varphi) = R[(1-1/V_R).\cos(\varphi) + (V_a /V_R).\cos((V_R-1). \varphi)]$$

$$y(\varphi) = R[(1-1/V_R).\sin(\varphi) - (V_a /V_R).\sin((V_R-1). \varphi)]$$

$\varphi$ : Winkel zum Zentrum des abrollenden Kreises (Kurvenparameter)
R : Radius des Festkreises
$V_R$ = R/r : Das Verhältnis zwischen dem Radius R des Festkreises und dem Ra lius r des abrollenden Kreises
$V_a$ = a/r : Das Verhältnis zwischen dem Abstand a des kurvenerzeugenden Punktes P vom Mittelpunkt des abrollenden Kreises und dem Radius r des abrollenden Kreises

3. Bei der erfindungsgemäßen Vorrichtung ist der Parameter $V_R$ ganzzahlig und hat Werte zwischen 3 und 8. Der Parameter $V_a$ hat Werte zwischen 0,1 und 0,9.
4. Die Schnittlinie in Form einer solchen Hypotrochoide wird dadurch erzeugt, dass mehrere kleinere, außenverzahnte Zahnräder entlang der Innenseite eines größeren, innenverzahnten Hohlzylinders abrollen.
5. Diese abrollenden Zahnräder sind ähnlich einem Planetengetriebe an einem rotierenden Halteteil drehbar befestigt.
6. Das größere, innenverzahnte Zahnrad steht still. Die abrollenden Zahnräder werden durch die Drehung des rotierenden Halteteiles in Bewegung gesetzt.
7. Das rotierende Halteteil besitzt eine Antriebsachse, die von einem externen Rotationsantrieb angetrieben wird und gegenüberliegend eine Zentrierachs die zur axialen Führung der Vorrichtung dient.
8. An jedem der abrollenden Zahnräder ist je ein Schneiddom befes igt. Alle Schneiddornen haben die gleiche Form und die gleichen Abmessungen.
9. Die Schneiddornen weisen bei bestimmten Ausführungsformen der erfindung igemäßen Vorrichtung an ihrer Vorderseite eine definierte Schneidfläche und Schneidkarne auf. Bei anderen Ausführungsformen sind an der Vorderseite Schleifmittel oder ein : Vielzahl von kleinen, raspelartigen Zacken angebracht.
10. Der Abstand zwischen der Mitte der Schneidkante auf den Schneiddornen und dem Mittelpunkt des kleiner Zahnrades, auf das diese jeweils montiert sind, entsoricht "a" des Parameters $V_a$ in Punkt 2 dieser Beschreibung. Die Winkel-Positionen der Schneiddornen auf den ihnen zugeordneten abrollenden Zahnrädern in Kombination mit ihren Eingriffs-Positionen in das größere, innenverzahnte Zahnrad ist für alle Schneiddornen so gewählt, dass alle Schneiddornen entlang der selben Hypotrochoide laufen. Die Schneiddornen erzeugen daher alle den selben Schnittkanal.

11. Die Anzahl der Zähne des größeren, innenverzahnten Zahnrades ist gleich den oder das Vielfache des kleinsten gemeinsamen Vielfachen von $V_R$, der Anzahl der abrollenden Zahnräder und der Anzahl der Zähne dieser abrollenden Zahnräder. Dadurch kann die relative Position der Schneiddomen auf jedem der abrollenden Zahnräder in Relation zur Verzahnung dieser Räder für alle abrollenden Zahnräder gleich sein.

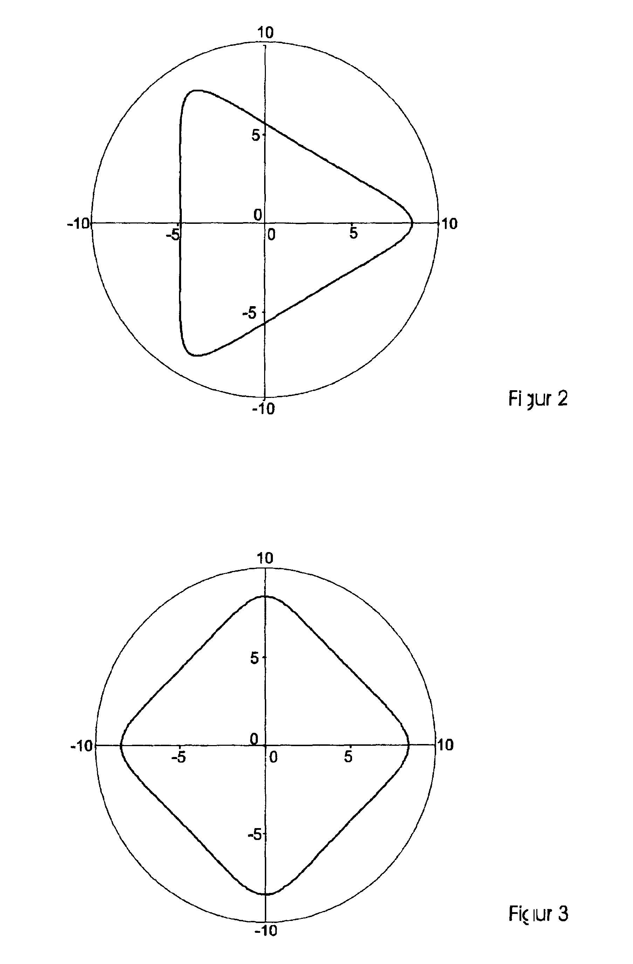

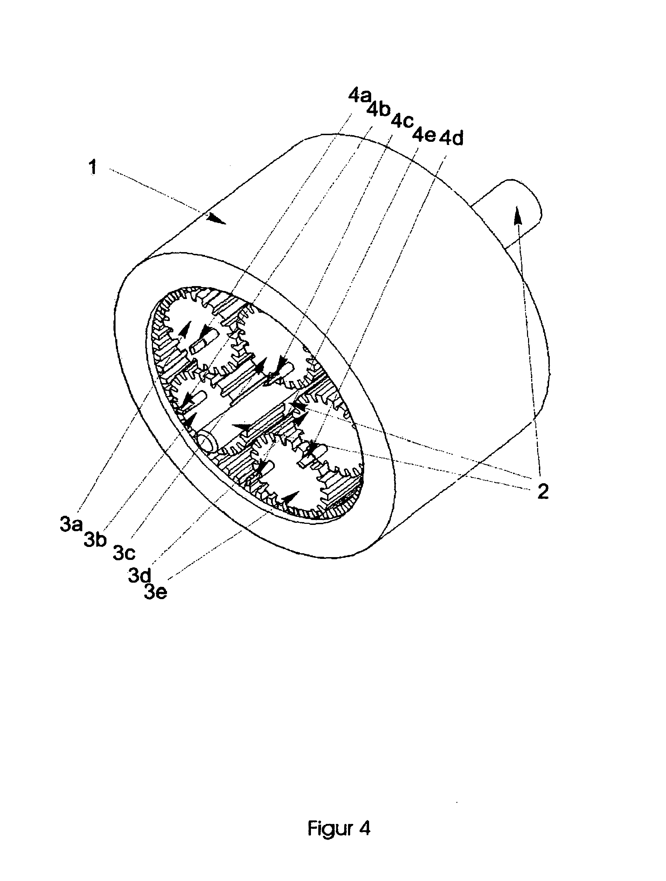

Die oben genannten Merkmale und vorteilhaften Eigenschaften des erfindungsgemäßen Vorrichtung werden aus den beiliegenden Zeichnungsfiguren noch deutlcher. Die Zeichnungsfiguren zeigen im einzelnen: Fig. 1 bis 3 zeigen Skizzen von Hypo rochoiden und Fig. 4 bi8 zeigen Ausführungsformen der erfindungsgemäßen Vorrichtung.

Figur 1 zeigt eine Skizze betreffend die Verhältnisse bei einer Hypotrochoide. Eine abrollende Kreisscheibe mit dem Radius r rollt ohne zu Gleiten im Inneren des estkreises R. Der kurvenerzeugende Punkt P ist Teil der abrollenden Kreisscheibe un liegt im Abstand "a" vom Mittelpunkt der abrollenden Kreisscheibe. Für die erfindur gsgemäße Vorrichtung ist a immer kleiner als r.

Wie bereits unter Punkt 2 der Beschreibung getan, sollen zwei Verhältnisse definiert werden:

$V_R$ = R/r : Das Verhältnis zwischen dem Radius R des Festkreises und dem Radius r des abrollenden Kreises

$V_a$ = a/r : Das Verhältnis zwischen dem Abstand a des kurvenerzeugenden Punktes vom Mittelpunkt des abrollenden Kreises und dem Radius r des abrollenden Kreises

Figur 2 zeigt ein Beispiel für eine Hypotrochoide, wie sie bei einer Ausführungsform der erfindungsgemäßen Vorrichtung verwendet wird; R =10, $V_R$ = 3 und $V_a$ = 11/20

Figur 3 Zeigt ein weiteres Beispiel für eine solche Hypotrochoide; R = 10, $V_R$ = 4 und $V_a$ =15/40

Figur 4 zeigt eine vorteilhafte Ausführungsform der erfindungsgemäßen Vorrichtung. In diesem Fall ist das Verhältnis $V_R$ gleich drei und die Anzahl der kleine Zahnräder ist fünf.

Ein einseitig geöffneter Hohlzylinder 1 ist an seiner Innenseite mit einer Verzahnung versehen. Er stellt das feststehende, innenverzahnte Zahnrad dar. Der Teilkreisdurchmesser der Innenverzahnung entspricht R, dem Radius des Fixkreises (siehe Punkt 2 der Beschreibung). An seiner geschlossenen Längsseite weist er eine Bohrung auf, durch die die hintere Achse eines rotierenden Trageteiles 2 gesteckt ist. Diese Achse ist bei bestimmten Ausführungsformen der erfindungsgemäßen Vorrichtung durch geeignete mechanische Elemente gegen axiale Verschiebung bezüglich des innenverzahnten Hohlzylinders gesichert, bei anderen Ausführungsformen ist der rotierende Tragteil in der Bohrung axial verschiebbar. Eine zweite vordere, Achse des rotierenden Trageteiles zentriert die erfindungsgemäße Vorrichtung in einem zuvor in den Knochen gebohrte Führungsbohrung.

Am rotierenden Trageteil 2 sind mehrere kleinere, außenverzahnte Zahnräder a, 3b, 3c, 3d, 3e drehbar befestigt. Ihre Verzahnungen greifen in die Innenverzal nung des Hohlzylinders 1 ein. Der Teilkreisradius dieser abrollenden Zahnräder 3a, 3b, 3c, 3d, 3e entspricht dem Wert "r" des abrollenden Kreises (siehe Punkt 2 der Beschreibur g).

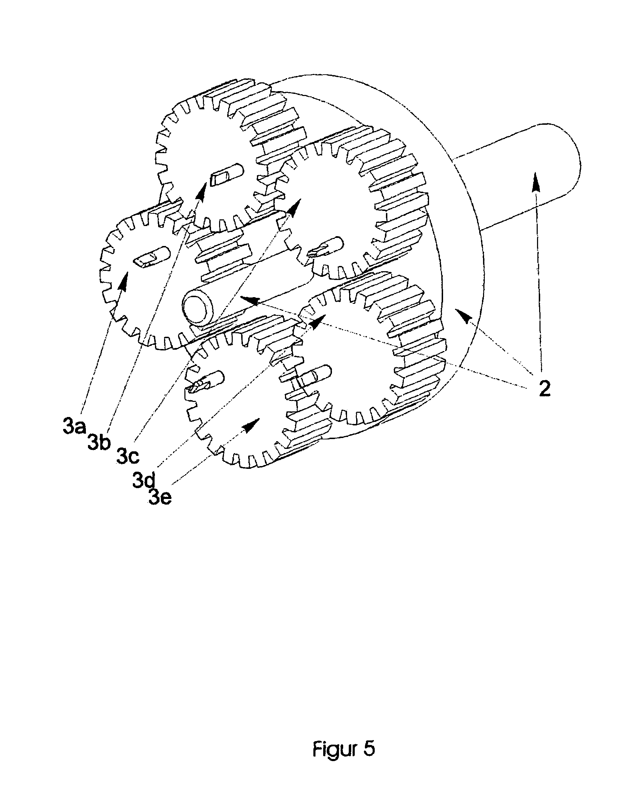

Jedes der kleinen Zahnräder weist eine Bohrung auf, in die jeweils ein Schneiddorn 4a, 4b, 4c, 4d, 4e eingepresst ist. beispiele für unterschiedliche Ausführungsformen des Schneiddorn sind in der Figur 5 dargestellt und werden dort genauer beschrieben.

Wenn die Achse des rotierenden Trageteiles 2 von einem externen Antrieb angetrieben wird, so rollen die kleinen Zahnräder 3a, 3b, 3c, 3d, 3e an der Innenverzal nung des Hohlzylinders 1 ab und die Schneiddornen 4a, 4b, 4c, 4d, 4e vollziehen eine Bahnbewegung in Form der durch die Parameter $V_R$ und $V_A$ bestimmten Hypotrochoide.

Für jeden Schneiddorn 4a, 4b, 4c, 4d, 4e und das entsprechende, abrollende Z hnrad 3a, 3b, 3c, 3d, 3e entspricht der Abstand zwischen der Mitte der Schneid kante des Schneiddorns und dem Mittelpunkt des abrollenden Zahnrades dem Wert "a" n Punkt 2 der Beschreibung und Figur 1. Die Winkelposition der Bohrung für die Schneindorne 4a, 4b, 4c, 4d, 4e auf dem abrollenden Zahnrädern 3a, 3b, 3c, 3d, 3e in Re ation zur Verzahnung diese Zahnrades ist für alle Schneiddornen bzw. abrollenden Zahlräder die selbe. Die Eingriffspositionen der Zähne der kleinen Zahnräder 3a, 3b, 3c, 3d 3e in die Innenverzahnung des Hohlzylinders 1 ist so gewählt, dass alle Schneiddornen 4a, 4b, 4c, 4d, 4e entlang der selben Hypotrochoide geführt werden und somit den selben Schnittkanal erzeugen; was aufgrund der oben beschriebenen Anzahl der Zähne der Innenverzahnung möglich ist.

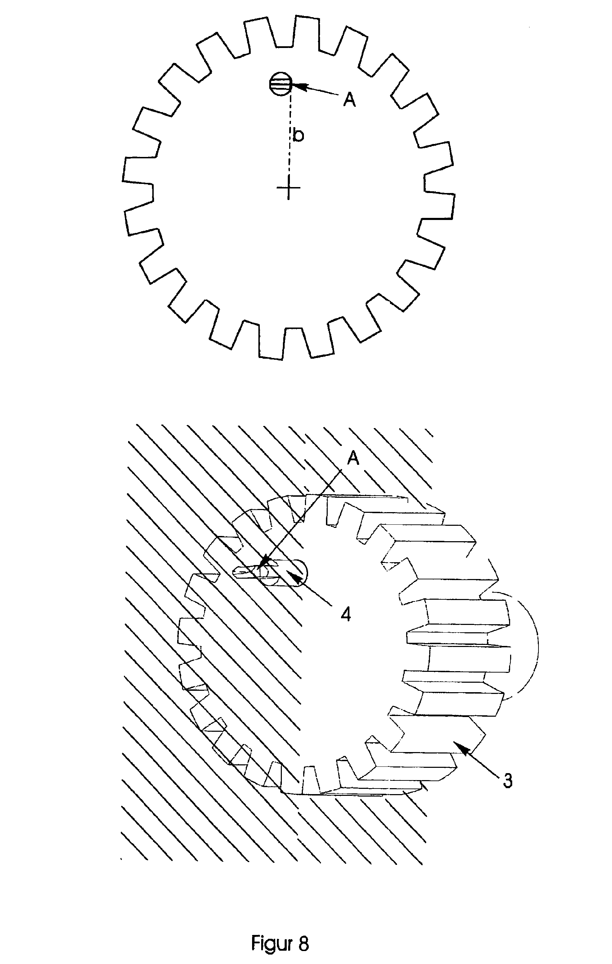

Alle Schneiddomen 4a, 4b, 4c, 4d, 4e haben die gleiche Gestalt und die gleichen Abmessungen. Sie sind jeweils so eingepresst, dass die Projizierenden ihrer Schneidflächen mit dem jeweiligen Radialstrahl vom Mittelpunkt des entspr chenden, abrollenden Zahnrades 3a, 3b, 3c, 3d, 3e zusammenfallen. Die Schneidflächen chneiden also immer rechtwinkelig zur Drehbewegung der kleinen Zahnräder. Dieser Sachverhalt ist in Figur 8 nochmals dargestellt.

Figur 5 zeigt den rotierenden Trageteil 2 mit den daran befestigten abrollende Zahnrädern 3a, 3b, 3c, 3d, 3e. Diese können sich am Trageteil um ihre Achsen frei drehen.

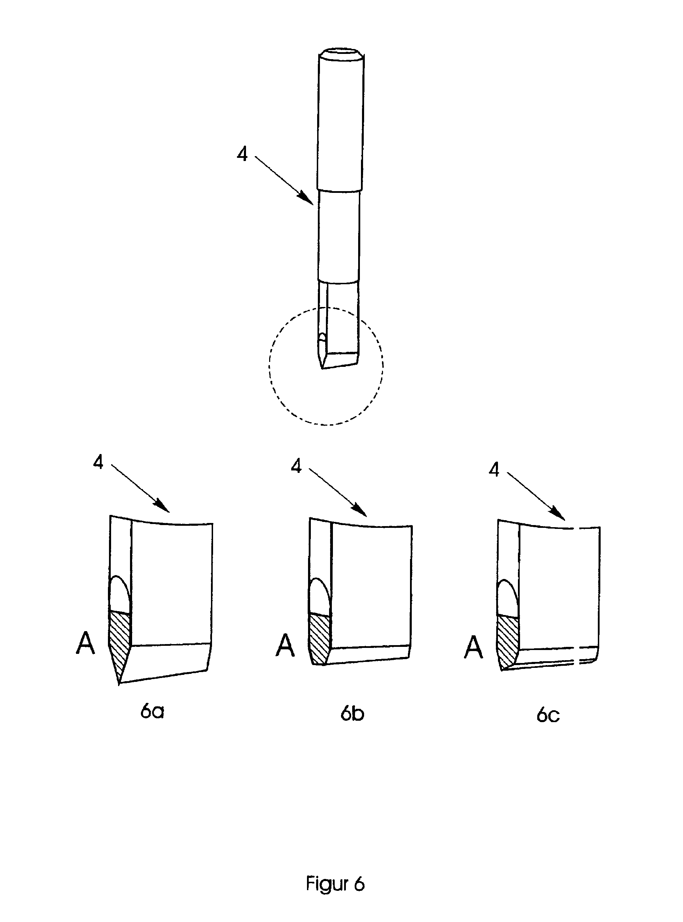

Figur 6 zeigt mehrere vorteilhafte Ausführungen des Schneiddornes) mit efinierter Schneidfläche und Schneidkante. Die Schneidgeometrie ist für Knochen optimiert. Bestimmte Ausführungsformen der Schneiddornen 4 haben eine scharfe Spitze, wie in Figur 6a dargestellt, andere Ausführungsformen besitzen eine gerade Schreidkante, entweder genau rechtwinkelig zur Achse des Schneiddorns (Fig. 6b) oder in einem bestimmten Winkel (Fig. 6c).

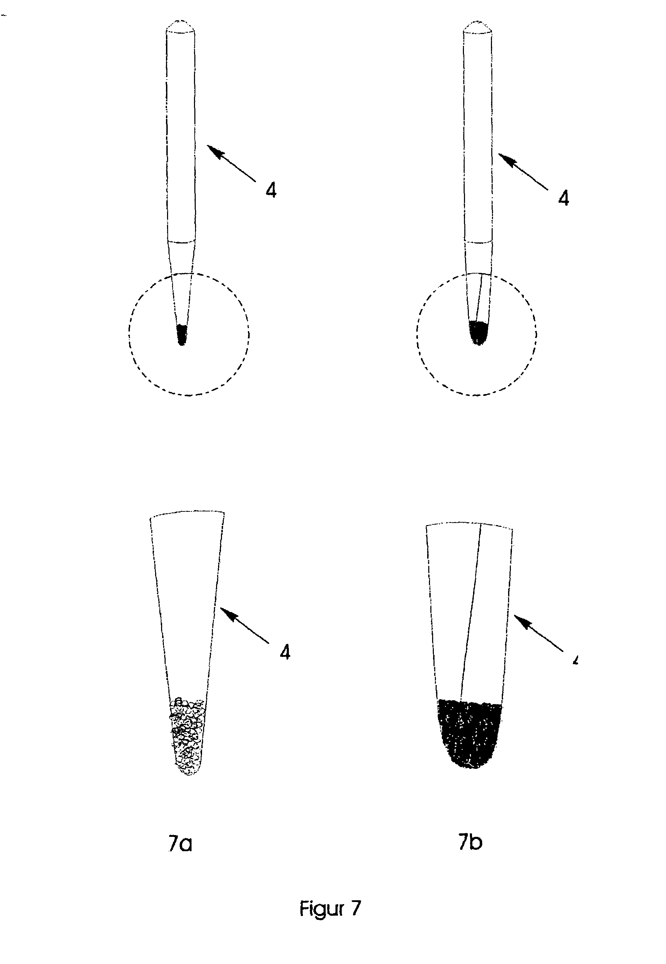

Figur 7 zeigt mehrere vorteilhafte Ausführungen des Schneiddornes 4 mit nicht definierten Schneidflächen. Die in Fig. 7a dargestellte Ausführungsform ist im vorderen Bereich mit Schleifmittel beschichtet. Die in Fig. 7b dargestellte Ausführungsform weist in vorderen Bereich eine Vielzahl von kleinen Zacken auf, die ähnlich einer Raspel den Schnittkanal erzeugen.

Figur 8 zeigt die genaue Position und Ausrichtung eines Schneiddornes 4 auf einem der abrollenden Zahnräder 3.

Die Winkelposition der Bohrung für den Schneiddorn ist in einem bestimmten Winkel zur Flanke eines Zahnes des abrollenden Zahnrades positioniert Dieser Winkel ist für alle abrollenden Zahnräder gleich.

Wenn man genau von axial auf das abrollende Zahnrad 3 blickt, dann kommt die Projizierende der Fläche A des Schneiddorns 4 genau mit dem Radialstrahl b zur Deckung. Der Abstand zwischen dem Mittelpunkt des kleinen Zahnrades und der Mitte der Schneidkante entspricht dem Abstand a des Parameters $V_a$ (siehe Punkt 2 der Beschreibung).

## Patentansprüche

1. Vorrichtung zur Anfertigung von Öffnungen in Knochen in Form eines Knochendeckels, **dadurch gekennzeichnet, dass** mehrere Schneiddornen (4a, 4b, 4c, 4d, 4e) entlar g ein und derselben Hypotrochoide geführt werden, deren Form in an sich bekannter Weise durch den Radius (R) des feststehenden Kreises, dem Radius des (r) des abrollenden Kreise und den Abstand (a) des kurvenerzeugenden Punktes (P) vom Mittelpunkt des abrollenden Kreises bestimmt ist.

2. Vorrichtung nach Anspruch 1, **dadurch gekennzeichnet, dass** das Verhältnis ($V_R$) zwischen dem Radius (R) des Festkreises und dem Radius (r) ($V_R = R/r$) des abrollenden Kreises ganzzahlig ist; insbesondere den Wert 3, 4, 5, 6, 7 oder 8 aufweist.

3. Vorrichtung nach Anspruch 2, **dadurch gekennzeichnet, dass** das Verhältnis ($V_a$) zwischen dem Abstand (a) des kurvenerzeugenden Punktes vom Mittelpunkt des abrollenden Kreises und dem Radius (r) des abrollenden Kreises ($V_a = a/r$) einen Wert zwischen 0.1 und 0,9 aufweist.

4. Vorrichtung nach Anspruch 2 oder 3, **dadurch gekennzeichnet, dass** die Anzahl der Schneiddornen (4a, 4b, 4c, 4d, 4e) je nach Ausführungsform einen Wert von 2 bis $2V_R$- 1 aufweist.

5. Vorrichtung nach einem der Ansprüche 1 bis 4, **dadurch gekennzeichnet, dass** der feststehende Kreis durch einen innenverzahnten Hohlzylinder realisiert ist.

6. Vorrichtung nach Anspruch 5, **dadurch gekennzeichnet, dass** in diesem innenverzahnten Hohlzylinder mehrere, außenverzahnte Zahnräder abrollen.

7. Vorrichtung nach Anspruch 6, **dadurch gekennzeichnet, dass** die Anzahl der ab aufenden Zahnräder (3a, 3b, 3c, 3d, 3e) einen Wert von 2 bis $2.V_R$ -1 aufweist.

8. Vorrichtung nach Anspruch 6 oder 7, **dadurch gekennzeichnet, dass** die at rollenden Zahnräder (3a, 3b, 3c, 3d, 3e) an einem rotierenden Trageteil (2) drehbar befestigt sind und durch die Drehbewegung dieses Trageteiles (2) an der Innenverzahnung des Hohlzylinders (1) abrollen.

9. Vorrichtung nach Ansprüchen 6 bis 8, **dadurch gekennzeichnet, dass** die Anzahl der Zähne der Innenverzahnung des Hohlzylinders (1) gleich ist dem oder ein Vielfaches des kleinsten, gemeinsamen Vielfachen des Wertes von $V_R$, der Anzahl der ab rollenden Zahnräder (3a, 3b, 3c, 3d, 3e) und der Anzahl der Zähne der abrollenden Zahnräder (3a, 3b, 3c, 3d, 3e).

10. Vorrichtung nach einem der Ansprüche 6 bis 9, **dadurch gekennzeichnet, dass** an jedem der abrollenden Zahnräder (3a, 3b, 3c, 3d, 3e) genau ein Schneiddorn (4a, 4b, 4c, 4d, 4e), vorzugsweise in einer Bohrung, befestigt ist.

11. Vorrichtung nach einem der Ansprüche 1 bis 10, **dadurch gekennzeichnet, dass** die Form und Abmessungen der Schneiddornen (4a, 4b, 4c, 4d, 4e) gleich ist.

12. Vorrichtung nach Anspruch 11, **dadurch gekennzeichnet, dass** jeder der Schneidornen (4a, 4b, 4c, 4d, 4e) an seiner Vorderseite eine definierte Schneidfläche und Schneidkante aufweist, wobei die Mitte der Schneidkante die Realisierung des kurvener: eugenden Punktes (P).ist

13. Vorrichtung nach Anspruch 11, **dadurch gekennzeichnet, dass** jeder der Schneidornen (4a, 4b, 4c, 4d, 4e) an seiner Vorderseite eine Vielzahl von nicht-definierten Schreidkanten aufweist, wobei die Mitte der Spitze des Schneiddornes (4a, 4b, 4c, 4d, 4e) die Realisierung des kurvenerzeugenden Punktes (P) ist.

14. Vorrichtung nach Anspruch 12 oder 13, **dadurch gekennzeichnet, dass** der A stand (a) zwischen dem kurvenerzeugenden Punkt (P) und dem Mittelpunkt des Zahnrades (3a, 3b, 3c, 3d, 3e), an dem der Schneiddorn (4a, 4b, 4c, 4d, 4e) befestigt ist, für alle al rollenden Zahnräder (3a, 3b, 3c, 3d, 3e) gleich ist.

15. Vorrichtung nach Anspruch 10, **dadurch gekennzeichnet, dass** die Winkelposition der Bohrung für die Schneidornen (4a, 4b, 4c, 4d, 4e) am entsprechenden Zahnrad (3a, 3b, 3c, 3d, 3e) in Relation zur Verzahnung dieses Zahnrades für alle abrollenden Zahnräder (3a, 3b, 3c, 3d, 3e) gleich ist.

16. Vorrichtung nach Anspruch 12, **dadurch gekennzeichnet, dass** die Schneidfläche jedes Schneiddornes (4a, 4b, 4c, 4d, 4e) so ausgerichtet ist, dass die Projizierende der Schneidfläche mit dem Zentralstrahl vom Mittelpunkt des entsprechenden Zahn ades (3a, 3b, 3c, 3d, 3e) zur Mitte der Schneidkante zusammenfällt.

17. Vorrichtung nach Anspruch 8 bis 16, **dadurch gekennzeichnet, dass** der notierende Trageteil (2) eine Achse zum Anschluß eines rotierenden Antriebes und dieser gegenüberliegend eine weitere Achse zum Zentrieren der erfindungsgemäßen Vorrichtung aufweist.

Figur 1

Fi gur 2

Fiç ıur 3

4a
4b
4c
4e 4d

1

3a
3b
3c
3d
3e

2

Figur 4

3a
3b
3c
3d
3e

2

Figur 5

Figur 6

7a

7b

Figur 7

Figur 8

Europäisches
Patentamt

European
Patent Office

Office européen
des brevets

# EUROPÄISCHER RECHERCHENBERICHT

Nummer der Anmeldung

EP 07 02 3956

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X<br>A | US 2004/034382 A1 (THOMAS JEFFREY E [US])<br>19. Februar 2004 (2004-02-19)<br>* Abbildungen 1-5 *<br>* Absätze [0011] - [0013], [0015] *<br>----- | 1-2,4,<br>11-13<br>3,5-10,<br>14-15 | INV.<br>A61B17/16 |
| A | DATABASE WPI Week 19815<br>Thomson Scientific, London, GB; AN<br>1981-A8958D<br>XP002567728<br>-& SU 737 200 A1 (CAR IND TECH RES INST)<br>30. Mai 1980 (1980-05-30)<br>* Zusammenfassung; Abbildung 1 *<br>----- | 1-15 | |
| A | DATABASE WPI Week 197840<br>Thomson Scientific, London, GB; AN<br>1978-H8124A<br>XP002567729<br>-& SU 580 064 A1 (MOSC AVIATION INST)<br>17. November 1977 (1977-11-17)<br>* Zusammenfassung; Abbildung 2 *<br>----- | 1-15 | |

RECHERCHIERTE
SACHGEBIETE (IPC)

A61B

Der vorliegende Recherchenbericht wurde für alle Patentansprüche erstellt

| Recherchenort | Abschlußdatum der Recherche | Prüfer |
|---|---|---|
| München | 10. Februar 2010 | Hochrein, Marion |

KATEGORIE DER GENANNTEN DOKUMENTE

X : von besonderer Bedeutung allein betrachtet
Y : von besonderer Bedeutung in Verbindung mit einer
anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
nach dem Anmeldedatum veröffentlicht worden ist
D : in der Anmeldung angeführtes Dokument
L : aus anderen Gründen angeführtes Dokument

    .................................................................
& : Mitglied der gleichen Patentfamilie, übereinstimmendes
Dokument

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.**

EP 07 02 3956

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

10-02-2010

| Im Recherchenbericht angeführtes Patentdokument | Datum der Veröffentlichung | Mitglied(er) der Patentfamilie | Datum der Veröffentlichung |
|---|---|---|---|
| US 2004034382    A1 | 19-02-2004 | AU    2003282050 A1<br>WO    2004014223 A2 | 25-02-2004<br>19-02-2004 |
| SU 737200        A1 | 30-05-1980 | KEINE | |
| SU 580064        A1 | 17-11-1977 | KEINE | |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 4029676 A1
- EP 0962192 A1
- GB 110179 A
- GB 8941420 A
- GB 1455566 A
- GB 2420979 A
- US 4768504 A1
- US 5201749 A1
- WO 9710765 A1